# EUROPÄISCHE PATENTSCHRIFT

(11) **EP 3 441 454 B1**
(45) Veröffentlichungstag und Bekanntmachung des Hinweises auf die Patenterteilung: **14.12.2022**
(21) Anmeldenummer: 18401066.8
(22) Anmeldetag: 30.07.2018
(51) Int. Cl.: C12M 1/107, C02F 11/12, C12M 1/00, C12M 1/02

(54) **VORRICHTUNG ZUR EINDICKUNG VON ORGANISCHEN SUBSTRATEN MIT EXTERNEM WÄRMETAUSCHER**
DEVICE FOR CONCENTRATION OF ORGANIC SUBSTRATES WITH EXTERNAL HEAT EXCHANGER
DISPOSITIF DE CONCENTRATION DES SUBSTRATS ORGANIQUES POURVU D'ÉCHANGEUR DE CHALEUR EXTERNE

(30) Priorität: 11.08.2017 DE 102017118356
(43) Veröffentlichungstag der Anmeldung: 13.02.2019
(73) Patentinhaber: Biogastechnik Süd GmbH, 88316 Isny im Allgäu (DE)
(72) Erfinder: Maier, Clemens, 88316 Isny-Sommersbach (DE); Maier, Gregor, 88316 Isny-Sommersbach (DE)
(74) Vertreter: Höchtl, Walter

(56) Entgegenhaltungen:
- EP-A1- 1 717 209
- EP-A1- 2 808 304
- EP-B1- 3 066 187
- DE-A1- 4 114 667
- DE-A1-102013 000 977
- DE-A1-102013 018 464
- DE-A1-102013 105 396
- DE-A1-102016 012 373

## Beschreibung

Die Erfindung bezieht sich auf eine Vorrichtung zur Eindickung von organischen Substraten mit einem Verdampfer der eine Einbringöffnung für das Substrat, einen Substrat - Auslass und einen oberen Auslass aufweist.

Herkömmliche Verdampfer zur Eindickung von organischem Substrat (Biomasse, Gülle, etc.) verfügen über eine integrierte Heizvorrichtung, die in der Regel als Wärmetauscher ausgebildet ist. Beim Erhitzen der Biomasse besteht das Problem, dass die Biomasse teilweise an der Heizvorrichtung anbackt und so die Wärmeübertragungsleistung gemindert wird. Zur Lösung dieses Problems beschreibt die EP3066187B1 einen Verdampfer, der mit einem reversierenden Bürstenmechanismus zur Reinigung der Heizvorrichtung ausgestattet ist. Diese Lösung ist zwar praktikabel aber technisch aufwendig.

Aus der DE 10 2013 000 977 A1 ist eine Anlage und ein Verfahren zur Trocknung von Gärresten bekannt, bei der ein Wärmetauscher einem Niedertemperaturverdampfer vorgeordnet ist. Die gesamte Anlage ist dabei hermetisch abgeschlossen und es wird in der gesamten Anlage, also auch im Wärmetauscher ein Unterdruck erzeugt.

Aus der EP 1 717 209 A1 ist ein Verfahren und ein System zur Behandlung von Schlämmen bekannt. Dabei ist einem Faulbehälter ein Wärmetauscher vorgeordnet. Der Schlamm wird mittels Pumpen durch den Wärmetauscher in den Faulbehälter verbracht. Im System können verschiedene Parameter mittels Sensoren gemessen werden und das System kann so gesteuert werden, dass die Viskosität des Schlammes gewünschten Vorgaben entspricht.

Aus der DE 10 2016 0123 73 ist eine Vorrichtung zum Aufschluss von biologischen Material bekannt. Die Vorrichtung weist einen Behandlungsraum zur Aufnahme des biologischen Materials als Dispersion und mindestens einen elektrischen Aktuator auf, der in der Dispersion Kavitation erzeugt. Die Vorrichtung umfasst weiter einen Behälter mit einer Einführöffnung und einer Austrittsöffnung zum kontinuierlichen Betrieb. Der Überstand über der Dispersion im Behälter kann mittels einer Vakuumpumpe evakuiert werden.

Aufgabe der Erfindung ist es, bei einer Vorrichtung zur Eindickung von organischen Substraten das Anbacken der organischen Substrate im Wärmetauscher zu vermeiden.

Diese Aufgabe wird mit einer Vorrichtung zur Eindickung von organischen Substraten dadurch gelöst, dass einem Verdampfer bzw. einem Verdampferkessel ein Wärmetauscher, vorgeordnet ist, an dessen Eingang eine Zuführpumpe und an dessen Ausgang ein Membran- oder ein Quetschventil, angeschlossen ist, wobei eine Steuerung oder Regelung für das Membran- oder das Quetschventilin Abhängigkeit vom Druck im Wärmetauscher vorgesehen ist, derart, dass im Wärmetauscher Normaldruck oder annähernd Normaldruck herrscht.

Nach einer vorteilhaften Ausbildung der Erfindung ist eine Steuerung oder eine Regelung für das Membran- oder das Quetschventil in Abhängigkeit von der Pumpleistung der Zuführpumpe (P1) ist.

Nach einer vorteilhaften Ausbildung der Erfindung ist an dem oberen Auslass des Verdampfers eine Vakuumpumpe angeschlossen. Dadurch wird im Verdampfer (-kessel) ein Vakuum erzeugt und somit die Verdampfung des Substrates verstärk.

Nach einer weiteren vorteilhaften Ausbildung der Erfindung ist zwischen der Vakuumpumpe und dem oberen Auslass ein Kondensator vorgesehen. Im Kondensator wird der Brüden abgekühlt und kondensiert, so dass die Vakuumpumpe ein geringeres und dichteres Volumen zu Pumpen hat.

Eine weitere vorteilhafte Ausbildung besteht darin, dass nach dem oberen Auslass des Verdampfers ein Brüdenwäscher angeordnet ist. Im Brüdenwäscher wird mittels Zugabe von Schwefelsäure das Ammoniak zu Ammoniumsulfat umgewandelt und aufkonzentriert. Die gewonnene Ammoniumsulfat - Lösung (ASL) kann als Dünger weiter verwendet werden. Das verbleibende Wasser kann z. Bsp. in die Kanalisation eingeleitet werden.

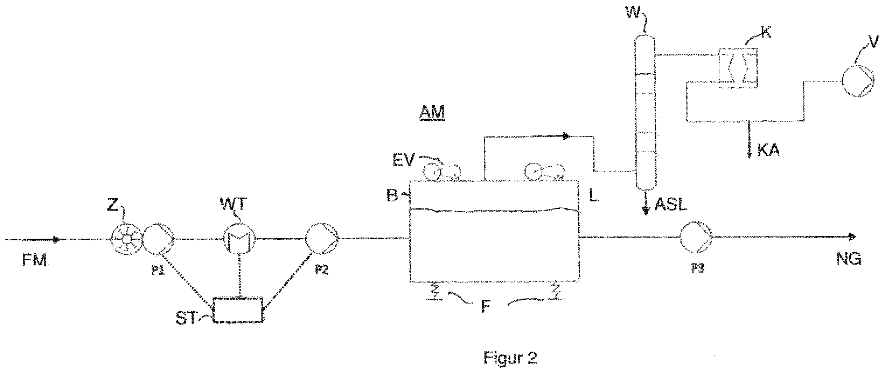

Nach einer weiteren vorteilhaften Ausbildung der Erfindung kann die Ausbeute an Biogas und ASL erheblich verbessert werden, wenn der Verdampfer - insbesondere im Ausführungsbeispiel gemäß Figur 2 - als Aufschlussmodul ausgebildet ist, das elektrische Aktuatoren (EV) zur Erzeugung von Kavitation im Substrat aufweist.

Ein Ausführungsbeispiel der Erfindung wird im Folgenden anhand der Figuren näher erläutert. Dabei zeigen die
- Figur 1: ein schematisches Schaltbild mit einem Verdampfer VD und einem externen Wärmetauscher WT
- Figur 2: ein schematisches Schaltbild mit einem Verdampfer VD und einem externen Wärmetauscher, wobei der Verdampfer als Aufschlussmodul ausgeführt ist.

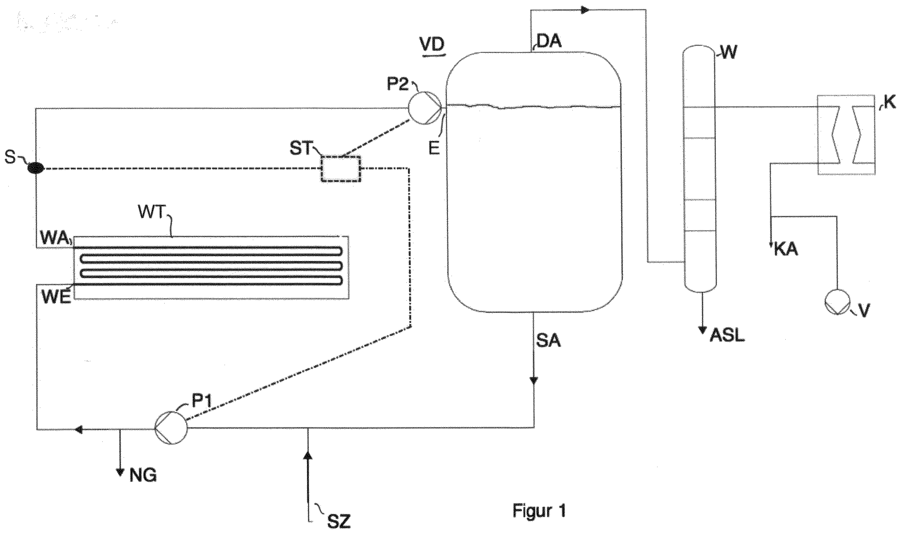

Die Darstellung gemäß Figur 1 zeigt einen Wärmetauscher WT, der als Rohrbündelwärmetauscher ausgeführt ist. Dieser ist mit seinem Eingang WE an den Ausgang der Zuführpumpe P1 angeschlossen. Der Ausgang des Wärmetauschers WA ist an ein Membran- oder Quetschventil P2 angeschlossen (der Einfachheit halber wird im Folgenden von einem Quetschventil ausgegangen), über das das Substrat in den Verdampfer VD über die Einbringöffnung E einspeist wird. Die Einbringöffnung E kann auch unten am Verdampfer VD angebracht sein. Über den Substrat-Auslass SA des Verdampfers VD wird das Substrat zur Zuführpumpe P1 zurückgeführt, mit neuem Substrat aus dem Substrat - Zulauf SZ gemischt und wieder - zumindest teilweise - über den Wärmetauscher und das Quetschventil P2 in den Verdampfer VD gespeist. Ein Teil des getrockneten Substrates kann nach der Zuführpumpe P1 über ein Ventil ausgeschleust und beispielsweise einem Nachgärer NG oder einem Gärrestelager GL zugeführt oder anderweitig gelagert oder verwendet werden.

Auf die Elemente Brüdenwäscher W, Kondensator K und die Vakuumpumpe wird später eingegangen. Zunächst wird die erfindungsgemäße Eindickung mit dem Wärmetauscher WT und dem Verdampfer VD beschrieben.

Das Quetschventil P2 und der Wärmetauscher WT sind mit einer Steuerung ST (oder Regelung) verbunden. (die elektrischen Komponenten sind gestrichelt dargestellt). Mittels eines Drucksensors S wird der Druck im Wärmetauscher WT zwischen seinem Ausgang WA und dem Eingang des Quetschventils P2 gemessen und das Quetschventil P2 wird so gesteuert oder geregelt, dass im Wärmetauscher WT Normaldruck oder annähernd Normaldruck herrscht. (Der Ausdruck "annähernd Normaldruck" ist hier so zu verstehen, dass je nach Qualität der Komponenten und der Steuerung auch kleine Druckschwankungen vorkommen können) . Dadurch kommt es auch nicht zur Bildung von Dampf im Wärmetauscher WT, so dass ein durch den Dampf bedingtes Anbacken von Substrat im Wärmetauscher WT, beispielsweise bzw. an den Rohren eines Rohrbündelwärmetauschers, unterbleibt.

Die Steuerung oder Regelung des Quetschventils P2 kann auch in Abhängigkeit von der Pumpleistung der Zuführpumpe P1 erfolgen. Dazu ist die Steuerung ST mit der Zuführpumpe P1 (strichpunktierte Linie) und dem Quetschventil P2 verbunden.

Das Membran- oder Quetschventil bremst in der Regel den Durchfluss. Es ist weniger anfällig gegen Verschmutzung oder Defekte als eine Pumpe und lässt sich ebenso über die Steuerung des Systems steuern oder regeln.

Beim Einbringen in den Verdampfer über das Quetschventil P2 entspannt sich das erhitzte Substrat und der Dampf trennt sich vom Substrat und kühlt auf die Dampftemperatur ab. Die über den Wärmetauscher WT zugeführte Wärmeenergie erhöht die Dampfproduktion und in Folge - als zusätzlichen Vorteil - die Produktion von ASL. Die Dampfproduktion wird zusätzlich durch die Erzeugung eines Unterdruckes im Verdampfer mittels der Vakuumpumpe V verstärkt.

Der entstehende Brüden kann nicht mehr von herkömmlichen bzw. ökonomisch sinnvollen Vakuumpumpen abgesaugt werden. Er muss vor der Vakuumpumpe V erst wieder gekühlt werden. Deshalb ist der Vakuumpumpe V ein Kondensator K vorgelagert, in dem der Brüden kondensiert und abgekühlt wird. Das kondensierte Wasser wird über die Leitung KA ausgebracht.

Je nachdem wie der Verdampfer betrieben wird, können auch geringere Dampfmengen am oberen Auslass anfallen, so dass anstelle eines Kondensators auch eine wassergekühlte Vakuumpumpe ausreichend wäre.

Mit einem dem Kondensator K vorgeschalteten Brüdenwäscher W wird das Ammoniak aus dem Brüden ausgewaschen und kann als ASL Dünger weiter verwendet werden.

Schließlich kann zwischen dem oberen Auslass DA und dem Brüdenwäscher W noch ein hier nicht näher dargestellter Abscheider vorgesehen werden, mit dem eventuelle Feststoffe aus dem Brüden ausgeschieden werden können.

In der Darstellung gemäß Figur 2 ist eine Variante der erfindungsgemäßen Vorrichtung mit einem Aufschlussmodul AM dargestellt. Das Substrat wird vom Fermenter über einen hier vorgesehenen Zerkleinerer Z und die bekannte Zuführpumpe P1 in den Wärmetauscher WT verbracht und über das Quetschventil P2 in das Aufschlussmodul AM gespeist. Der Zerkleinerer ist nur bei entsprechender Substrat - Konsistenz notwendig. Wird beispielsweise Gülle verarbeitet, ist er nicht notwendigerweise vorzusehen.

Die Steuerung bzw. Regelung der Pumpe P1 und des Quetschventils P2 erfolgt wie oben beschrieben, ebenso die Absaugung des Brüden durch die Vakuumpumpe V über den Brüdenwäscher W und den Kondensator K.

Das Aufschlussmodul AM besteht unter anderem aus dem luftdichten Behälter B, der nur bis zum Level L mit Substrat gefüllt ist. Im Hohlraum oberhalb des Substratfüllstandes L wird der Brüden über den oberen Auslass DA und die Vakuumpumpe V abgesaugt.

Am Behälter B des Aufschlussmoduls AM sind zwei elektrische Aktuatoren, hier zwei elektromagnetische Vorrichtungen EV, angebracht. Diese bringen das Substrat mittelbar über den Behälter B in vertikale Schwingungen, so dass im Substrat Kavitationsblasen entstehen. Um die Schwingungen zu ermöglichen ruht der Behälter auf vier gefederten Standbeinen. Verschiedene Vorrichtungen zur Erzeugung von Kavitation in einem solchen Behälter sind aus dem Stand der Technik bekannt.

Das Substrat wird aus dem Behälter mittels der Pumpe P3 ausgeleitet und kann in einen Nachgärer NG verbracht werden, wo durch das aufgeschlossene Substrat die Biogasproduktion optimiert wird. Substratzu- und -abfluss von und zum Aufschlussmodul AM können auch kontinuierlich erfolgen.

Durch den externen Wärmetauscher kann das Substrat bei höheren Temperaturen im Vergleich zu herkömmlichen Verdampfern verdampft werden.

## Patentansprüche

1. Vorrichtung zur Eindickung von organischen Substraten mit einem Verdampfer (VD) der eine Einbringöffnung (E) für das Substrat, einen Substrat - Auslass (SA) und einen oberen Auslass (DA) aufweist,
**dadurch gekennzeichnet, dass,** dem Verdampfer (VD) ein Wärmetauscher (WT), vorgeordnet ist, an dessen Eingang (WE) eine Zuführpumpe (P1) angeschlossen ist, und an dessen Ausgang (WA) ein Membran- oder ein Quetschventil (P2) angeschlossen ist, wobei eine Steuerung (ST) oder eine Regelung für das Membran- oder das Quetschventil (P2) in Abhängigkeit vom Druck im Wärmetauscher (RW) vorgesehen ist, derart, dass im Wärmetauscher (WT) Normaldruck oder annähernd Normaldruck herrscht.

2. Vorrichtung zur Eindickung von organischen Substraten nach Anspruch 1,
**dadurch gekennzeichnet, dass**, eine Steuerung (ST) oder eine Regelung für das Membran- oder das Quetschventil (P2) in Abhängigkeit von der Pumpleistung der Zuführpumpe (P1) vorgesehen ist.

3. Vorrichtung nach einem der vorstehenden Ansprüche, **dadurch**
**gekennzeichnet, dass,** an dem oberen Auslass (DA) des Verdampfers (VD) eine Vakuumpumpe (V), angeschlossen ist.

4. Vorrichtung nach Anspruch 3, **dadurchgekennzeichnet, dass**, zwischen der Vakuumpumpe (V) und dem oberen Auslass (DA) ein Kondensator (K) vorgesehen ist.

5. Vorrichtung nach einem der vorstehenden Ansprüche, **dadurch**
**gekennzeichnet, dass** nach dem dem oberen Auslass (DA) des Verdampfers (VD) ein Brüdenwäscher (W) angeordnet ist.

6. Vorrichtung nach einem der vorstehenden Ansprüche, **dadurch gekennzeichnet, dass** der Verdampfer als Aufschlussmodul ausgebildet ist, das elektrische Aktuatoren (EV) zur Erzeugung von Kavitation im Substrat aufweist.

## Claims

1. Device for thickening organic substrates with an evaporator (VD) having an inlet (E) for the substrate, a substrate outlet (SA) and an upper outlet (DA), **characterized in that** the evaporator (VD) is preceded by a heat-exchanger (WT) arranged upstream of the evaporator (VD), to the inlet (WE) of which a feed pump (PI) is connected, and a diaphragm or pinch valve (P2) is connected to its outlet (WA), wherein a control (ST) or a regulation for the diaphragm valve or the pinch valve (P2) is provided in dependence on the pressure in the heat exchanger (WT), in such a way that normal pressure or approximately normal pressure prevails in the heat exchanger (WT).

2. Device for thickening organic substrates according to claim 1, **characterised in that** a control system (ST) or a regulation system for the diaphragm valve or the pinch valve (P2) is provided as a function of the pumping capacity of the feed pump (PI).

3. Apparatus according to one of the preceding claims, **characterized in that** at the upper outlet (DA) of the evaporator (VD), a vacuum pump (V) is connected.

4. Device according to claim 3, **characterised in that**, between the vacuum pump (V) and the upper outlet (DA) a condenser (K) is provided.

5. Device according to one of the preceding claims, **characterized in that** downstream of the upper outlet (DA) of the evaporator (VD) a vapour scrubber (W) is arranged.

6. Apparatus according to one of the preceding claims, **characterized in characterised in that** the evaporator is in the form of a digestion module, which has electrical actuators (EV) for generating cavitation in the substrate.

## Revendications

1. Dispositif de concentration des substrats organiques pourvu d'un évaporateur (VD) comportant une ouverture d'introduction (E) pour le substrat, une sortie de substrat (SA) et une sortie supérieure (DA),
**caractérisé en ce qu**'un échangeur de chaleur (WT) est agencé en amont de l'évaporateur (VD), à l'entrée (WE) duquel est raccordée une pompe d'alimentation (P1), et à la sortie (WA) duquel est raccordée une soupape à membrane ou une soupape d'écrasement (P2), dans lequel une commande (ST) ou un réglage est prévu pour la soupape à membrane ou la soupape d'écrasement (P2) en fonction de la pression dans l'échangeur de chaleur (RW), de telle sorte qu'il y ait une pression normale ou approximativement normale dans l'échangeur de chaleur (WT).

2. Dispositif de concentration des substrats organiques selon la revendication 1, **c aractérisé en ce qu**'une commande (ST) ou un réglage est prévu pour la soupape à membrane ou la soupape d'écrasement (P2) en fonction de la puissance de pompage de la pompe d'alimentation (P1).

3. Dispositif selon l'une quelconque des revendications, **caractérisé en ce qu**'une pompe à vide (V) est raccordée à la sortie supérieure (DA) de l'évaporateur (VD).

4. Dispositif selon la revendication 3, **caractérisé en ce qu**'un condensateur (K) est prévu entre la pompe à vide (V) et la sortie supérieure (DA).

5. Dispositif selon l'une quelconque des revendications précédentes,
**caractérisé en ce qu**'un laveur de brouillards (W) est agencé en aval de la sortie supérieure (DA) de l'évaporateur (VD).

6. Dispositif selon l'une quelconque des revendications précédentes,
**caractérisé en ce que** l'évaporateur est formé en tant que module de dissolution comportant des actionneurs électriques (EV) pour la création de cavitation dans le substrat.
